Europäisches Patentamt

European Patent Office

Office européen des brevets

(11) Publication number:

0 303 364

A1

## (12) EUROPEAN PATENT APPLICATION

(21) Application number: 88306779.5

(22) Date of filing: 22.07.88

(51) Int. Cl.⁴: **C12N 15/00**

(30) Priority: 10.08.87 CA 544122

(43) Date of publication of application:
15.02.89 Bulletin 89/07

(84) Designated Contracting States:
AT CH DE FR GB IT LI SE

(71) Applicant: **The Governors of the University of Alberta**
**1-3 University Hall**
**Edmonton Alberta T6G 2J9(CA)**

(72) Inventor: **Jensen, Susan E.**
**8808 - 187 Street**
**Edmonton Alberta, T6G 2E9(CA)**
Inventor: **Westlake, Donald W. S.**
**12604 - 62 Avenue**
**Edmonton Alberta, T6G 1N5(CA)**
Inventor: **Leskiw, Brenda K.**
**No. 300, 10149 Saskatchewan Drive**
**Edmonton Alberta, T6E 6B6(CA)**
Inventor: **Aharonowitz, Yair Dep. of Mibrobiology**
**Faculty of Life Sciences Tel Aviv University**
**Ramat Aviv 69-978 Tel Aviv(IL)**
Inventor: **Mevarech, Mosheir Dep. of Mibrobiology**
**Faculty of Life Sciences Tel Aviv University**
**Ramat Aviv 69-978 Tel Aviv(IL)**

(74) Representative: **Bond, Bentley George et al**
**Haseltine Lake & Co. 28 Southampton Buildings Chancery Lane**
**London WC2A 1AT(GB)**

(54) Cloning and nucleotide sequence determination of the isopenicillin N synthetase gene from streptomyces clavuligerus.

(57) The gene coding for the enzyme Isopenicillin N Synthetase (IPNS) isolated from the prokaryote Strep-tomyces clavuligerus is cloned and sequenced. The corresponding amino acid sequence is also determined. The enzyme IPNS controls the rate of synthesis of the β-lactam family of antibiotics. The nucleotide sequence is incorporated into a vector to effect a transformation of a suitable host microorganism. Such a transformed microorganism is useful in making in vitro synthesis of the β-lactams more practical and in whole cell fermentations to produce higher levels of β-lactams.

## CLONING AND NUCLEOTIDE SEQUENCE DETERMINATION OF THE ISOPENICILLIN N SYNTHETASE GENE FROM STREPTOMYCES CLAVULIGERUS

FIELD OF THE INVENTION

This invention relates to the cloning and sequencing of the Isopenicillin N synthetase (IPNS) gene from the prokaryote Streptomyces clavuligerus for use in the production of $\beta$-lactam compounds.

BACKGROUND OF THE INVENTION

The $\beta$-lactam family includes penicillins, cephalosporins, cephamycins and clauvulanic acid which are important antibiotics. Of the many naturally occurring compounds, only Penicillin G and Clavulanic acid are used directly in medicine. The other clinically important $\beta$-lactam compounds are derived by structural changes to the naturally occurring compounds. Most recent methods involve nuclear modification of the natural $\beta$-lactam products. Such modifications involve complex chemical processes consisting of numerous steps. The products are obtained in a low yield and at high cost.

Recently much attention has been focussed on manipulating the natural production of $\beta$-lactam compounds. Several microorganisms including the prokaryote Streptomyces clavuligerus produce a variety of $\beta$-lactam compounds. The enzymes which comprise the biosynthetic pathway of the $\beta$-lactams have been studied in some detail. A schematic representation of this pathway appears in Diagram 1.

```
L-α-aminoadipic acid     ACV synthetase
+ L-cysteine                |
+ L-valine                  |
                            ↓

δ-(L-α-aminodiapyl)-L-cystenyl-D-valine (ACV)

                   |        Isopenicillin N Synthetase
                   ↓
          Isopenicillin N
                   |
                   |        Epimerase
                   ↓

          Penicillin N
                   |
                   |        Expandase
                   ↓

          Desacetoxycephalosporin C
                   |
Cephalosporin C  ↖  ↓
                      ↘
                        Desacetylcephalosporin C
Cephamycin C  ↙
```

After hydroxylation of the cephalosporin, a number of different products may be produced depending on the microorganism. For example, in S. clavuligerus, the hydroxylation is followed by a carbamoylation, another hydroxylation and methylation resulting in a cephamycin.

Several of the individual enzymes have been isolated and studies with substrate analogues have demonstrated the ability of the enzyme system to synthesize both natural and unnatural penicillins and cephalosporins in cell-free systems. Similar studies have been conducted on the corresponding enzymes from the eukaryotic, cephalosporin C producing fungus, Cephalosporium acremonium. For example, United States patent 4,178,210 issued December 11, 1979 to A.L. Demain et al teaches the conversion of the D-

2

form penicillin N to a cephalosporin compound by contacting the precursor with a cell-free extract of C. acremonium. In United States patent 4,307,192 issued December 22, 1981, Demain et al concentrate on enzymes at a later stage in the biosynthetic pathway namely the epimerase and expandase isolated from a cell-free extract of C. acremonium.

The limitations of the above two patents is their reliance on enzymes isolated from C. acremonium. Although similarities between the enzyme system of C. acremonium and S. clavuligerus are observed as expected, in view of the identical chemical reactions that they catalyze, considerable differences are also apparant. Most notable, the epimerase, expandase and hydroxylase enzymes show differences in stability and physical properties. The enzymes isolated from S. clavuligerus and more stable, and exhibit greater activity than those of C. acremonium and are therefore more useful in cell-free systems.

## SUMMARY OF THE INVENTION

According to an aspect of the invention, a nucleotide sequence codes for the enzyme, isopenicillin N synthetase which is a protein produced by the metabolic system of Streptomyces clavuligerus.

According to another aspect of the invention, a vector having the nucleotide sequence is inserted therein.

According to another aspect of the invention, a biologically pure culture of a microorganism is transformed with the vector.

According to another aspect of the invention, a process for preparing the enzyme, isopenicillin N synthetase comprises culturing the microorganism transformed with the vector and isolating the produced enzyme from the culture.

According to another aspect of the invention, an amino acid sequence of the isopenicillin N synthesis enzyme is provided.

## BRIEF DESCRIPTION OF THE DRAWINGS

Preferred embodiments of the invention are shown in the drawing wherein:

Figure 1 is a restriction map and strategy for sequencing the isopenicillin N synthetase gene; and

Figure 2 is the nucleotide sequence of the isopenicillin N synthetase gene and the amino acid sequence of isopenicillin N synthetase. In each row of the respective sequences, the upper row is a listing by standard nomenclature of the nucleotide sequence. The lower row in each respective row of sequences is standard nomenclature for the amino acid corresponding to the respective codon.

## DETAILED DESCRIPTION OF THE PREFERRED EMBODIMENTS

The prokaryote S. clavuligerus is a well known microorganism which is readily available and is on deposit and may be obtained under accession number ATCC #. Although this prokaryote has been extensively studied, no attempts has been made to isolate in the microorganism the gene or genes encoding for isopenicillin N synthetase. The genes isolated from C. acremonium and Penicillium chrysogenum, which encoded for the IPNS gene of those eukaryotes, could not be used as probes in locating the corresponding gene or genes in the prokaryote S. clavuligerus.

The antibiotic synthesizing enzymes make up a small proportion of the total protein in wild type strains of microorganisms and antibiotic production by these strains is correspondingly low. The naturally occurring low levels of these enzymes coupled with the attendant difficulty of purifying usable quantities of enzymes has made in vitro synthesis of antibiotics commercially impractical. If greater quantities of these antibiotic synthesizing enzymes could be produced, in vitro synthesis would be more practical. Similarly, whole cell fermentations might yield higher levels of antibiotics if biosynthetic enzyme levels could be increased in the producer organism.

It has been recognized that Isopenicillin N Synthetase plays a key role in determining the rate of the β-lactam biosynthetic pathway. The IPNS level limits the output of the penicillin/cephalosporin pathway. Consequently by increasing the levels of the IPNS, it would be possible to increase the production of the β-lactams. The possible method to increase the levels of IPNS would be to clone the gene coding for IPNS and insert multiple copies of this gene into the genome which would result in high levels of expression of the same gene.

3

A particular gene coding for IPNS has been isolated from C. acremonium sequenced and expressed in Escherichia coli (Samson et al, Isolation, Sequence Determination, and Expression in E. Coli of the Isopenicillin N synthetase Gene from C. acremonium, Nature 318, pp 191-194 1985). The IPNS gene has also been used as a heterologous probe to isolate the IPNS gene from Penicillium chrysogenum. However, the two distinct genes isolated from these eukaryotes are inferior to that of the prokaryote S. clavuligerus. As mentioned earlier the enzymes coded for by the prokaryotic S. clavuligerus gene are more stable and exhibit better activity than its eukaryotic counterparts. Furthermore, the antibiotic synthesizing genes may be clustered together on the S. clavuligerus genome. This clustering would have many advantages in genetic manipulations.

According to this invention, it has been discovered that a single gene codes for the IPNS of S. clavuligerus. The gene was isolated by developing a DNA probe based on an amino acid sequence isolated from the purified IPNS removed from S. clavuligerus. The probe was used to select from a genomic library of S. clavuligerus a DNA sequence by way of hybridization. By way of restriction mapping transformation into a microorganism, it was discovered that the identified DNA sequence contained the gene encoding the IPNS.

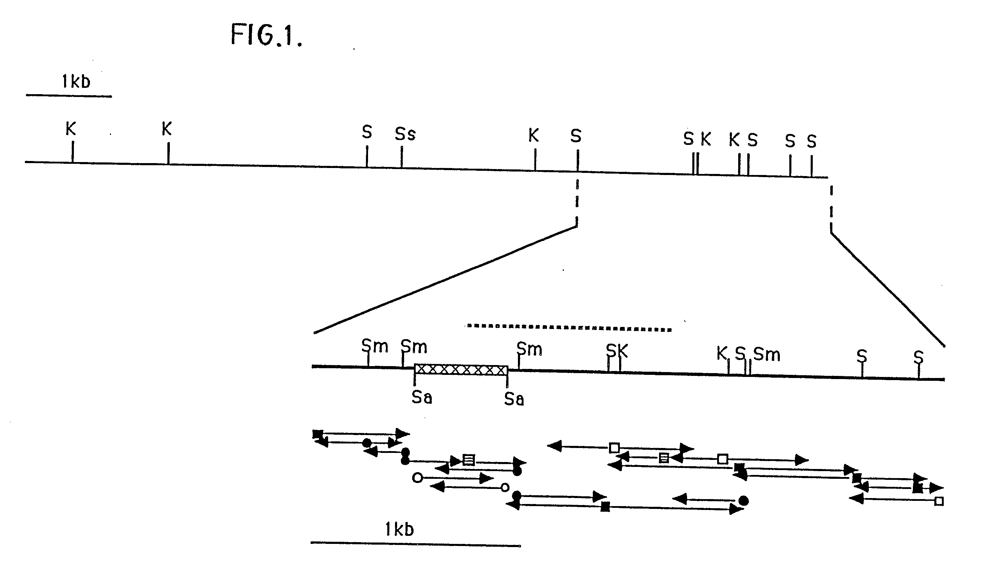

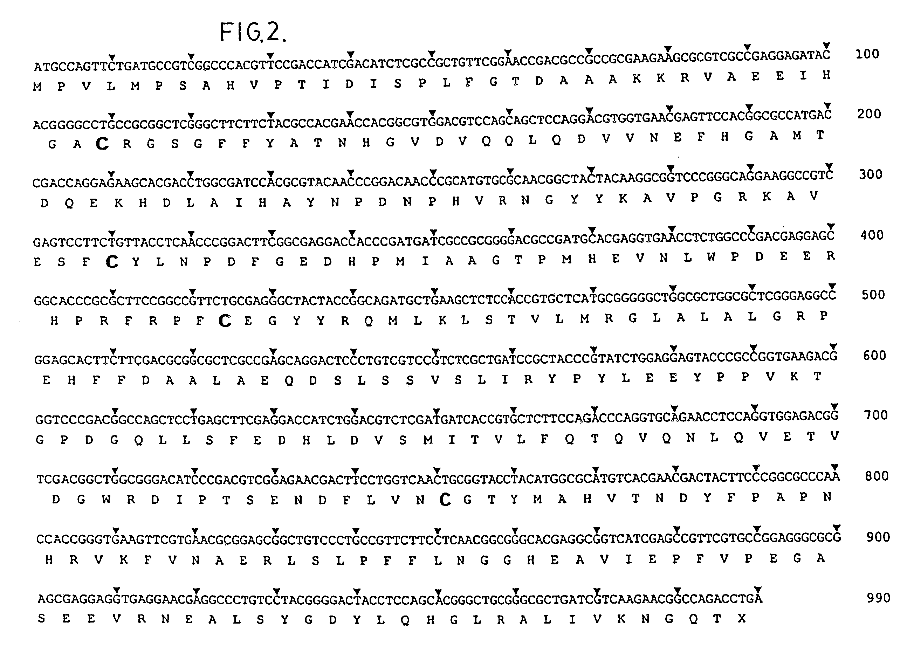

As shown in Figure 1, the restriction mapping and strategy in locating and sequencing the IPNS gene is demonstrated. The identified clone pBL1, which was isolated from the partial Sau3A library is represented by the thin line. The region of DNA that was sequenced is represented by the bold line shown in large in the lower part of the Figure 2. The dotted line represents the complete IPNS gene. The Sau3A fragment, which hybridized with the oligonucleotide probe, is shown by the cross-hatched box. Arrows below the map indicate the direction and extent of the sequence information determined for each fragment. Both strands were sequenced for 100% of the IPNS coding region and all restriction endonuclease junctions were overlapped. In Figure 1, the legend is as follows:

K-Kpn1, s = Sal1, Sa-Sau3A, Sm = Sma1, Ss = Sst1 ■ □ ○ ● ⊟ = Sal1, Kpnl, Sau3A, Smal and specific oligonucleotide primer sequence start points respectively.

Figure 2 shows the nucleotide sequence of the IPNS gene taken from the pBL1. In accordance with standard nomenclature, in each numbered row 100 to 990, the upper level of each row is a depiction of the nucleic acid sequence, where in accordance with standard nomenclature, the letters A, C, G, and T stand for the known nucleic acids adenine, cytosine, guanine and thyamine. In each row the lower level includes a series of capital letters indicating each of the amino acids corresponding to a codon of the DNA sequence. The DNA sequence as shown is from the translation start site to the termination codon X. The amino acid sequence of the enzyme can be predicted from the discovered DNA sequence. The numbers at the ends of the rows refer to the base pair locations and the arrows above the lines denote every ten base. The amino acids are represented by standard nomenclature where letters in bold type indicate positions for cysteine residues. The nomenclature for the amino acids is as follows:

A = alanine, R = arginine, N = asparagine, D = aspartate, C = cysteine, E = glutamate, Q = glutamine, G = glycine, H = histidine, I = isoleucine, L = leucine, K = lysine, M = methionine, F = phenylalanine, P = proline, S = serine, T = threonine, W = tryptophan, Y = tyrosine, V = valine and X-stop codon.

Accordingly, this invention has isolated the nucleotide sequence which codes for the enzyme IPNS of the particular prokaryote S. clavuligerus. This gene may be inserted in a suitable vector for transforming a desired microorganism which, when transcribed, is capable of producing the enzyme. Such vectors may be in the form of plasmids, bacterial phages and viral phages.

The transformed microorganism with the vector may be in the form of a biologically pure culture. The microorganism, when cultured in a suitable medium, produces the enzyme IPNS. The enzyme may be then isolated from the culture for use in synthesizing both natural and unnatural penicillins and cephalosporins in cell-free systems. Alternatively, several copies of the gene may be inserted by a suitable vector system in the prokaryote to enhance production in vivo of the enzyme and hence, expedite the overall production of the desired antibiotic.

In sequencing the DNA for the IPNS gene, the amino acid sequence of the enzyme is now determined. Such information leads to a better understanding of the structure of the enzyme and its active sites.

The following examples exemplify the manner in which the IPNS gene of S. clavuligerus was discovered and the manner in which they may be transformed in a microorganism to produce the enzyme.

EXAMPLE 1: PURIFICATION OF IPNS

## Preparation of Cell-Free Extracts

Forty eight hour cultures of <u>S. clavuligerus</u> grown on trypticase soy broth (Baltimore Biological Laboratories) containing 1% (w/v) soluble starch (Difco) [TSBS] were harvested by filtration through Whatman No. 2 filter paper and washed with 1 L of 0.05 M Tris-HCl buffer, pH 7.2 containing 0.1 mM DTT and 0.01 mM EDTA (TDE buffer). Washed cells were resuspended in 200 ml of TDE contained 10 μM trans-epoxysuccinyl-L-leucylamido-(4-guanidino)-butane (E-64 from Sigma Chemicals). Cell-free extracts were prepared by sonication of washed cell suspensions for 2 x 15 seconds at intensity setting 7 (Sonifier Cell Disrupter 350, 0.75 inch diameter probe, Branson Sonic Power Co.). Broken cell suspensions were centrifuged for 15 minutes at 27,000 xg.

## Streptomycin Sulfate/Ammonium Sulfate Precipitation

Solid streptomycin sulfate was gradually added to cell-free extract with gentle stirring at 4° C to a final concentration of 1% (w/v). After 15 minutes at 4° C, the suspensions were centrifuged for 15 minutes at 17,000 xg, and the pellet was discarded. Solid ammonium sulfate was then added to the supernatant as described above and that material precipitating between 50% and 70% saturation collected by centrifugation. The pellet was resuspended in 10 ml of TDE buffer containing 10 μM E-64 was desalted by passage through a Sephadex (trademark) G-25 column (2.5 x 35 cm) which has been equilibrated in TDE buffer.

## Isocratic Ion Exchange (DEAE-trisacryl) Chromatography

Desalted enzyme extract from the previous step was applied to a DEAE-trisacryl column (1.6 x 30 cm) which had been equilibrated with TDE buffer containing 0.125 M Tris-HC1, pH 7.2, instead of 0.05 Tris-HC1, pH 7.2. The column was eluted with 150 ml of 0.125 M TDE buffer. Fifty drop (2.5 ml) fractions were collected and eluant was monitored for absorbance at 280 nm with a Uvicord SII(LKB). The IPNS activity was located by assaying 0.03 ml amounts of each fraction using the bioassay methods.

## Assays

## Isopenicillin N Synthetase (IPNS) Assay

The conversion of ACV (from Penninsula Laboratories) to isopenicillin N was measured in reaction mixtures containing: 0.287 mM bis-ACV, 4 mM dithiothreitol (DTT), 2.8 mM sodium ascorbate, 45 μM FeSO$_4$, 0.05 mM Tris HCl buffer (pH 7.2) and enzyme to give a final volume of 0.04 ml (Jensen, S.E. et al Purification of Isopenicillin N synthetase from <u>Streptomyces clavuligerus</u>, <u>Can. J. Microbiol.</u> 32: 953-958, 1986a). Reaction mixtures were incubated for 10 to 60 minutes at 20° C and terminated by the addition of 0.4 ml of methanol. Product formation was measured by HPLC analysis (Jensen, S.E. et al, Cyclization of δ-(L-α-aminoadipyl)-L-cysteinyl-D-valine to Penicillins by Cell-Free Extracts of <u>Streptomyces clavuligerus J. Antibiot.</u> 35, pp 483-490 1982a) or by agar diffusion bioassays using <u>Micrococcus luteus</u> ATCC 9341 (Jensen et al, <u>supra</u> and Jensen, S.E. et al, Cephaslosporin Formatin cy Cell-Free Extracts from <u>Streptomyces clavuligerus, J. Antibiot.</u> 35, pp 1531-1360 1928b).

## Gradient Elution Ion Exchange (DEAE-trisacyl) Chromatography

IPNS-containing fractions from the isocratic DEAE-trisacryl chromatography (in 0.125 M TDE buffer) were pooled and diluted with 0. 1 mM DTT in 0.01 mM EDTA to give a final Tris-HCl concentration of 0.05 M. This enzyme preparation which had thus been adjusted to contain normal TDE buffer was applied to a DEAE-trisacryl column (1.6 x 30 cm) which had been equilibrated with TDE buffer. The column was washed with 50 ml of TDE buffer and then eluted with a linear Tris-HCl gradient composed of 200 ml of TDE versus 200 ml of TDE containing 0.2 M Tris-HCl (pH 7.2). Fifty drop (2.5 ml) fractions were collected and eluant was monitored for absorbance at 280 nm with a Uvicord SII(LKB). The IPNS activity was located by assaying 0.03 ml amounts of each fraction using the bioassay method.

**Gel Filtration (Superose 12 HR 10/30) Chromatography**

IPNS-containing fractions from the DEAE-trisacyl chromatography were pooled and concentrated to a final volume of 2.0 ml by ultrafiltration using a PM-10 filter (Amicon Corporation). The entire 2.0 ml enzyme concentrate was further purified by repeated application of 0.2 ml aliquots (maximum allowable sample volume) to a Superose 12 HR 10/30 column attached to the following HPLC equipment: models 6000 and 45 pumps; model UK-6 injector; model 490 variable wavelength detector; model 680 gradient controller (all from Waters Scientific Co.). Data were recorded on a Hewlett Packard 3390A integrator/recorder. The column was equilibrated and eluted with TDE buffer at a flow rate of 0.5 ml/min and 0.5 ml fractions were collected using a Gilson Model 201 fraction collector. Activity was located as described for the DEAE-trisacryl chromatography.

**Ion Exchange (Mono-Q HR 5/5) Chromatography**

Enzyme-containing fractions from all of the Superose 12 columns were pooled and the entire pool was applied to a Mono Q HR 5/5 column attached to the same HPLC equipment described previously. The Mono Q column was equilibrated with 0.02 M Tris-HCl buffer (pH 7.2) containing 0.1 mM DTT, 0.01 mM EDTA and 0.1 M KCL at a flow rate of 1 ml/min. After sample application, the column was washed with he equilibration buffer for 2 min and then eluted with a 20 min linear gradient starting with the equilibration buffer and ending with the same buffer but containing 0.27 M KCL. One milliliter fractions were collected and 0.03 ml amounts were assayed for IPNS activity.

The purified IPNS was sent to an amino acid sequencing service (Dr. R. Olafson, University of Victoria) where amino acid sequence information was obtained for the thirty five amino acid residues at the amino terminus. The sequence information obtained was as follows:

NH₂-pro-val-leu-met-pro-ser-ala-his-val-pro-thr-ile-asp-ile-ser-pro-
    1                5                   10                  15

leu-phe-gly-thr-asp-ala-ala-ala-lys-lys-arg-val-ala-glu-glu-ile-his-gly-ala
        20                  25                  30                  35

EXAMPLE 2: PREPARATION OF OLIGONUCLEOTIDE PROBES

The amino terminal amino acid sequence information was used to deduce the corresponding DNA sequence which would give rise to such a peptide. Taking into consideration the biased codon usage which has been shown to occur in Streptomyces species, a 44-mer oligonucleotide probe was designed which hybridizes specifically with the IPNS gene. The probe was synthesized by the Regional DNA Synthesis Laboratory, University of Calgary, and has the following sequence:

5'-CCGTGGATCTCCTC(CG)GC(CG)AC(CG)CGCTTCTT
       5    10    15        20        25

(CG)GC(CG)GC(CG)GCGTC(CG)GT-3'
    30        35        40

The probe sequence corresponds to amino acids 20 through 34 of the amino terminal amino acid sequence. Bases given in brackets indicate positions where mixtures of bases were employed. The probe codes for the complementary strand of DNA and contains 128-fold degeneracy.

## EXAMPLE 3: KINASE LABELLING OF OLIGONUCLEOTIDE PROBE

The oligonucleotide probe (0.5 $\mu$g) of Example 2 is labelled with 100 $\mu$Ci[$\gamma^{32}$P]dATP using T4 polynucleotide kinase according to the recommendations of the manufacturer (Pharmacia). The labelled probe is separated form the unincorporated [$\gamma^{32}$P]dATP by gel filtration chromatography on a 10 ml Sephadex G-50 column. The probe is further purified by filtration through a disposable syringe filter unit (Millex HA, 0.45 $\mu$m).

## EXAMPLE 4: PREPARATION OF A GENOMIC DNA CONTAINING THE IPNS GENE

Streptomyces clavuligerus genomic DNA was isolated using a procedure based on that of Hopwood et al, Genetic Manipulation of Streptomyces - A Laboratory Manual, The John Innes Foundation, Norwich, U.K. The cells were grown as described above and harvested by filtration through Whatman No. 2 filter paper. One gram (wet weight) of mycelium was resuspended in 5 ml of 10 mM Tris-HCl, pH 8.0, containing 1 mM EDTA (TE buffer) in a 50 ml screw cap tube. Lysozyme was added to a final concentration of a 2 mg/ml and swirled to dissolve. The lysozyme mixture was incubated at 30°C until a drop of suspension was cleared upon addition of a drop o 10% SDS. A solution of EDTA (0.5 M, pH 8.0) was added to give a final concentration of 0.12 M, mixed gently, and then pre-digested pronase solution (10 mg/ml in water: pre-digested by incubation at 30°C for 10 minutes) was added to give a final concentration of 0.2 mg/ml. This was mixed gently and incubated at 30°C for 5 minutes. Sodium dodecyl sulfate was added to give a final concentration of 1% (w/v), the tube was tilted immediately to mix and the solution was incubated at 37°C until complete clearing was achieved (1 to 2 hours). An equal volume of equilibrated phenol (500 g melted phenol, 0.5 g 8-hydroxyquinoline, and 65 ml of TE buffer containing 0.1 M NaCl) was added and mixed by thorough shaking for 10 minutes at 21°C. The same volume of chloroform was added and mixed by shaking. The suspension was then centrifuged at 1000 xg for 10 minutes at room temperature and the aqueous phase was carefully transferred with a shortened pasteur pipette to a fresh screw cap tube. Five milliliters of TE buffer containing 0.1 M NaCl was added to the phenol phase, mixed by shaking and centrifuged as above. This aqueous phase was then combined with the aqueous phase obtained from the first extraction and the pooled aqueous phases were re-extracted with equilibrated phenol and chloroform as described above. Traces of phenol were removed by extraction of the aqueous phase with 2 x 6 ml of chloroform, and the aqueous phase was transferred to a pre-weighed tube. The tube plus sample was weighted and DNase-free RNase [1 mg/ml in 20 mM Tris-HCl, pH 7.5, containing 1 mM MgSO$_4$, 100 mM NaCl and 0.01% (w/v) gelatin] was added to give a final concentration of 40 $\mu$g/g of DNA solution.

After incubation at 37°C for 1 hour, 0.25 ml of 5 M NaCl was added and mixed, and then 30% PEG 6000 was added to a final concentration of 10% (w/v). The solution was mixed gently until the DNA precipitated (about 1 minute) and the DNA was then spooled onto a glass rod, transferred to a fresh tube and dissolved in 5 ml of TE buffer. When the DNA was completely dissolved, 0.6 ml 3 M sodium acetate and 12 ml 98% ethanol were added and mixed. The DNA, which precipitated immediately, was spooled onto a glass rod and transferred to a fresh tube where it was washed with 2 ml of 70% (v/v) ethanol. The ethanol was removed; the DNA was dissolved in 2 ml of sterile TE buffer and stored at 4°C.

## EXAMPLE 5: PARTIAL DIGESTION OF GENOMIC DNA CONTAINING THE IPNS GENE

The isolated genomic DNA of Example 4 was then partially digested with Sau3A using a procedure based on that of Maniatis et al (Molecular Cloning - A Laboratory Manual, Cold Spring Harbour Laboratory, Cold Spring Harbour, New York, 1982). Fragments of an average size of 10 kb were produced by digesting 220 $\mu$g of S. clavuligerus DNA with Sau3A at a final concentration of 0.8 units of enzyme/$\mu$g DNA in buffer. After digestion, the DNA was extracted first with an equal volume of phenol and then twice with n-butanol to remove traces of phenol. The digested DNA was then precipitated by addition of 2 volumes of 98% ethanol and redissolved in 0.2 ml of TE buffer containing 160 mM NaCl. The restriction fragments are purified by preparative sucrose gradient centrifugation. To prepare a fraction, which was enriched with 10 kb fragments, gradients containing 12 ml of 5 to 20% sucrose in TE buffer plus 160 mM NaCl are prepared to cooled to 4° C. The DNA solution was carefully layered on top of the gradients (0.2 ml/12 ml gradient) and the gradients were centrifuged at 80,000 x g for 14 hours at 4° C. Immediately after centrifugation, the gradients were fractionated by repeatedly removing 0.4 ml aliquots from the top using an Eppendorf pipette. The DNA was precipitated with 98% ethanol as above, dissolved in 30 $\mu$l TE and stored at -20° C.

## EXAMPLE 6: S. CLAVULIGERUS GENOMIC LIBRARY CONSTRUCTION

Two micrograms of pUC19 (ATCC 37254) was digested with BamHI, treated with calf intestinal alkaline phosphatase and then extracted twice with an equal volume of phenol/chloroform (v/v). This digested plasmid preparation was then extracted once with an equal volume of chloroform, and precipitated with 98% ethanol as described above. The vector was redissolved in distilled water at a final concentration of 0.1 $\mu$g/$\mu$l.

The Sau3A digested genomic DNA preparation of Example 5 enriched for fragments of about 10 kb was ligated, using a standard procedure (Maniatis et al, Molecular Cloning - A Laboratory Manual, Cold Spring Harbor Laboratory, Cold Spring Harbor, New York, 1982) into the BamHI site of pUC19 using an insert to vector ratio of 2:1 and a final DNA concentration 0.038 $\mu$g/$\mu$l. The ligated recombinant plasmid preparation was transformed into E. coli JM109 competent cells according to the recommendations of Strategene Cloning Systems except the transformed cells were plated of MacConkey plates supplemented with ampicillin instead of LB/X-gal/ampicillin plates.

Master plates were prepared by transferring colonies containing recombinant plasmids (lactose negative) onto LB agar plates supplemented with ampicillin in an ordered array to form a library of S. clavuligerus DNA fragments in pUC19.

## EXAMPLE 7: SCREENING OF LIBRARY COLONIES BY COLONY HYBRIDIZATION

Libraries of Example 6 were screened by colony hybridization. Twenty-seven nitrocellulose filter reproductions, each representing 96 colonies, were prepared from the master plates and, after prehybridization, were exposed ot the oligonucleotide probe which had been end labelled with [$\gamma$32P]dATP. The nitrocellulose filters were washed free of unbound radioactivity under high stringency conditions and bound radioactivity was visualized by autoradiography.

Filter reproductions of the ordered master plates were prepared by placing nitrocellulose filters (Millipore HAFT, 0.45 $\mu$m) on LB agar plus ampicillin plates, transferring the colonies with sterile toothpicks from the master plates to the nitrocellulose filter plates and incubating overnight at 37° C. The filters were then removed from the plates and treated to lyse the colonies and bind the liberated DNA as described by Maniatis et al (Supra) except that after neutralization the filters were soaked in 0.3 M NaCl containing 0.03 M sodium citrate (2 x SSC) and the colony debris was removed by gentle rubbing.

Each filter was placed in a heat-sealable, plastic bag, and then 3 ml of prehybridization solution [ 30% (v/v) formamide, 6 x SSC, 1X Denhardt's 0.1% (w/v) SDS, 50 $\mu$g/ml sonicated salmon sperm DNA (Sigma)] was added. The bags were sealed after air is removed and then incubated at 55° C for 2 hours.

Hybridization was performed by replacing the prehybridization solution with 25 ml of fresh prehybridization solution containing about 4 $\mu$Ci of 32p-labelled probe. The filters were hybridized overnight at 55° C and then washed twice in 6 x SSC and twice in 2 X SSC solutions containing 0.1% (w/v) SDS and 30% (v/v) formamide for 1 hour at 55° C. The filters were then air dried and autoradiographed (-70° C) using Kodak X-OMAT AR film. One heavily labelled spot which corresponded to a colony, was observed. This plasmid bearing clone was designated pBL1.

## EXAMPLE 8: HOMOLOGY OF ISOLATED DNA FRAGMENT TO PROBE

Homology to the probe was confirmed by digesting individual samples of plasmid DNA from pBL1 with the 6-base specific restriction endonucleases EcoR1, HindIII, Pst1, Sst1, Sal1, Sph1, Sma1, and Kpn 1. The digests are electrophoresed on a 1% agarose gel and the separated DNA fragments transferred to nitrocellulose (Schleicher and Schuell, Inc.) by the method of E. Southern (Detection of Specific Sequences among DNA Fragments Separated by Gel Electrophoresis, J. Mol. Biol. 98, pp 503,517, 1975), as described by Maniatis et al (Supra) Prehybridization, hybridization, washing and autoradiography is carried out as described above.

The smallest fragment visible on the autoradiogram that hybridized with the oligonucleotide probe was 1.4 kb Sal1 fragment. The 1.4 kb hybridizing Sal1 fragment was subcloned by shotgun cloning Sal1 fragments of pBL1 into the Sal1 site of pUC19 and transforming E. Coli JM109 with the mixture. The appropriate subclone is identified by colony hybridization as described above.

The subclone was analyzed further by digestion with the 4-base specific restriction endonucleases Msp1, Taq1, Sau3A, Cfo1, and Alu1. The DNA fragments were separated by agarose gel electrophoresis, transferred to nitrocellulose and hybridized as described above. Autoradiography indicated that a Sau3A fragment of about 400-500 bp hybridized to the probe.

The 450 bp hybridizing Sau3A fragment was electroeluted onto DEAE membrane (NA-45, Schleicher and Schuell, Inc.) using the band interception method (Lizardi et al 1984) and the DNA was purified from the membrane according to the manufacturer's specifications. The fragment was then ligated into the BamH1 site of pUC119, transformed into E. coli JM109 and the appropriate subclone was identified by colony hybridization. The DNA fragment was then transferred from the pUC19 vector to M13mp 19 as an EcoR1/HindIII fragment.

The fragment was then sequenced by the chain termination method (Sanger, F.S. et al, DNA Sequencing with Chain-Terminating Inhibitors, Proc. Natl. Acad.Sci USA, y4, pp 5463-5467, 1977). Inspection of the resulting nucleotide sequence revealed a stretch of the sequence which corresponded to the predicted sequence used to generate the oligonucleotide probe. One base of the probe sequence was incorrect due to the choice of C in position 24 of the probe sequence. The actual gene had an A in that position. Both alternatives were correct in terms of the amino acid which would result, since ACA and ACC both code for alanine as required by the amino acid sequence. Furthermore, the nucleotide sequence immediately surrounding the probe area also correspond to the known amino acid sequence for the amino terminal part of the IPNS protein.

## EXAMPLE 9: RESTRICTION MAPPING OF pBL1

The plasmid pVL1 was digested with Sal1, Kpn1, Sst1 and Sma1 singly and in double digests, to generate a restriction map of the entire pBL1 plasmid and to locate the IPNS gene of the insert. The restriction map is shown in Figure 1. No restriction sites were found for EcoR1, BamH1, HindIII, BglII, Pst1 or Nco1. Fine mapping of the region containing the IPNS gene (shown enlarged in the lower part of Figure 1) was achieved by isolating subclones containing relevant Sal1, Sst1 and Kpn1 fragments from pBL1 by shotgun cloning or by cloning electroeluted DNA. Appropriate subclones were identified after plasmid isolation. These subclones were mapped after digestion with Sau3A, Kpn1, Sal1 and Sma1. The size of the entire S. clavuligerus DNA fragment which was inserted in pBL1 was found to be about 9.6 kb.

**Restriction Fragment Analysis**

**Polyacrylamide Gel Electrophoresis**

DNA fragments of 75 to 700 bp were subjected to electrophoresis on 5% (w/v) polyacrylamide gels using a TEA buffer system (40 mM Tris-acetate, pH 8.0, containing 1 mM EDTA). Appropriate molecular weight markers were HinF1 fragments of pBR322.

**Agarose Gel Electrophoresis**

DNA fragments in the range of 1 to 10 kb were subjected to electrophoresis in 1% agarose gels using the TEA buffer system described above. Appropriate molecular weight markers were BstEII fragment of λ phage.

## EXAMPLE 10: DNA SEQUENCE ANALYSIS

DNA sequencing is conducted by the chain termination method of Sanger et al (Supra). In order to conduct DNA sequencing, Sal1, Kpn1 and Sma1 fragments shown in the enlarged lower part of Figure 1, were subcloned into pUC19 (Vieira, J. et al, The pUC Plasmids, an M13mp7-Derived System for Insertion Mutagenesis and Sequencing with Synthetic Universal Primers, Gene, 19, pp 258-268, 1982).

Subclones containing the Sal1, Kpn 1, Sma1 and Sau3A fragments shown in the enlarged lower part of Figure 1 were isolated by shotgun cloning or by cloning electroeluted DNA as described above. All DNA fragments subcloned in pUC119 were transferred to M13mp18 and M13mp19 as EcoR1/HindIII fragments and sequenced by the dideoxy method of Sanger et al (Supra). The start point, direction of sequencing and extent of sequence information obtained from each M13 subclone is shown by the arrows in Figure 1. The entire nucleotide sequence of the gene and its translation to amino acid codons is shown in Figure 2. Both strands were sequenced for the entire protein-coding region which spans 987 bp and codes for a protein of 329 amino acids.

In order to conduct a DNA sequencing, appropriate small DNA fragments of pBL1 of Example 7 were subcloned into pUC19 (Vieira et al, Supra) either by a shotgun method or after electroelution from polyacrylamide gels. Competent cells of E. coli JM109 were transformed as described above. Appropriate transformants were identified by restriction analysis of plasmid DNA, isolated by a modification of the method of Birnboim et al, (A Rapid Alkaline Extraction Procedure for Screening Recombinant Plasmid DNA, Nucl. Acids Res., 7, pp 1513-1523, 1979) on either polyacrylamide or agarose gels. Once the pUC19 subclones of interest were identified, the inserts were transferred from pUC19 into the M13 vectors mp 18 and mp 19 (Yanisch-Peron et al, Improved M13 Phage Cloning Vectors and Host Strains: Nucleotide Sequences of the M13mp18 and pUC19 Vectors, Gene, 33 pp 103-119, 1985). Competent E. coli JM109 cells were then transfected with the recombinant M13 vectors. Single stranded M13 template DNA are then prepared as recommended by International Biotechnologies Inc, for their rapid deletion cloning system.

Dideoxy sequencing reactions were conducted using the universal M13 sequencing primer and all reactions employed [$\alpha^{32}$P]dATP as the radioactive tracer as described by Sanger et al (Supra). Labelled fragments produced in the sequencing reactions were separated electrophoretically of 6 or 8% polyacrylamide (38:2 acrylamide:N,N-methylene bisacrylamide)- 8.3 M urea gels using a TEB buffer system (60 mM Tris-HCl, 1 mM EDTA, 60 mM borate). Compressions in the banding pattern were relieved by analyzing sequencing reaction mixtures on gels containing 7 M urea and 40% (v/v) formamide (Martin, R, Overcoming DNA Sequencing Artifacts: Stops and Compressions, BRL Focus, 9, pp 8-9 1987). In addition compressions were also relieved using a "sequenase" sequencing kit which contains reaction mixtures with dITP in place of dGTP (Barnes et al, Kilo-Sequencing: Creation of an Ordered Nest of Asymmetric Deletions Across a Large Target Sequence Carried on Phage M13, Methods of Enzymology, 101, pp 98-122, Eds.R. Wu, L. Grossman and K. Moldave, Academic Press, New York, 1983).

Visualization of radioactive bands in sequencing gels was performed by autoradiography (-20 °C) using Kodak X-OMAT AR film.

## EXAMPLE 11: PROTOPLAST FORMATION AND TRANSFORMATION OF IPNS GENE INTO ANTIBIOTIC NON-PRODUCING MUTANTS OF S. clavuligerus

Streptomyces clavuligerus NTG 1 is a mutant of S. clavuligerus NRRL 3585 which was produced in our laboratory by N-methyl-N -nitro-nitrosoguanidine mutagenesis. The mutant does not produce detectable amounts of cephamycin C and appears to be specifically defective in IPNS products. Protoplasts of S. clavuligerus NTG 1 were prepared using a procedure based on that of D.A. Hopwood et al (Genetic Manipulation of Streptomyces - A Laboratory Manual. The John Innes Foundation, Norwich, U.K. 1985). All procedures were carried out under aseptic conditions. Cultures (20 ml) were harvested by centrifugation for 15 min at 27,000 x g at 21 °C and washed with 10 ml of 0.3 M sucrose. The washed cells were resuspended in 5 ml of 0.3 M sucrose, homogenized briefly and diluted with 5 ml of 0.3 M sucrose (tissue grade size 23, Kontes Glass 6). The cell suspensions were centrifuged for 10 minutes as described above. The mycelium was resuspended in 4 ml of lysozyme solution (1 mg/ml lysozyme dissolved in P buffer of the following compositions: 0.5 M sucrose, 0.57 mM $K_2SO_4$, 48 mM $CaCl_2$, 25 mM MOPS, pH 7.2, 0.59 $\mu$M $ZnCl_2$, 1.48 $\mu$M $FeCl_3$, 0.12 $\mu$M $CuCl_2$, 0.10 mM $MnCl_2$, 0.52 $\mu$M $Na_2B_4O_7$, 0.16 $\mu$M $(NH_4)_6Mo_7O_{24}$, 1% (w/v) bovine serum albumin) and was incubated for 15 minutes at 28 °C with gentle swirling at 50 rpm. The solution was triturated three times with a 5 ml pipette before and after the addition of 5 ml of P buffer. The solution was filtered through non-adsorbent cotton into fresh screw cap tubes. The protoplasts were pelletted by centrifugation for 10 minutes at 1,500 x g and washed with 5 ml of P buffer. The protoplasts are resuspended in P buffer and counted using a hemacytometer (Bright-Line, AO Scientific Instruments Inc.).

A 1 ml aliquot, containing 2.5 x 10$^9$ protoplasts were centrifuged as described above and the pellet is resuspended in a drop of P buffer remaining after the supernatant was decanted. The protoplasts were then transformed by mixing them with 1.5 μg ligated plasmid DNA, In the presence of 0.5 ml of T buffer [P buffer containing 25% (w/v) PEG 1000 in place of bovin serum albumin]. The ligated plasmid DNA was constructed by combining, in a 2:1 ratio, pBL1 and pIJ941, each digested with EcoR1 and Pst 1. The transformation mixture was pipetted gently to mix the incubated at room temperature for 30 seconds before the addition of 5 ml P buffer. The transformed protoplasts were centrifuged for 10 minutes at 1,500 x g and plated on generation agar; 0.3 M sucrose, 0.58 mM K$_2$SO$_4$, 5.9 mM sodium glutamate, 68 mM CaCl$_2$, 25 mM MOPS, pH 7.2, 0.20 mM MgSO$_4$, 0.29 μM ZnCl$_2$, 0.74 μM FeCl$_3$, 0.059 μM CuCl$_2$, 0.051 μM MnCl$_2$, 0.26 μM Na$_2$B$_4$O$_7$, 0.008 μM (NH$_4$)$_6$Mo$_7$O$_{24}$, 1%(v/v) glycerol, 0.1% (w/v) Difco Casamino acids, 0.5% (w/v) yeast extract containing 2% (w/v) agar (R2YE) and overlayered at 40 hours with R2YE soft agar [0.6% (w/v) agar containing thistrepton at a final concentration of 5 μg/ml]. Colonies bearing recombinant plasmids were identified by lack of growth of YEME containing 1% glycerol, 5 μg/ml thiostrepton and 200 μg/ml hygromycin B due to insertional inactivation of the hygromycin resistance gene.


EXAMPLE 12: EXPRESSION OF IPNS ACTIVITY IN ANTIBIOTIC NON-PRODUCING MUTANTS OF S. clavuligerus

The entire 9.6 kb insert of S. clavuligerus DNA was removed form pBL1 by digestion with EcoR1 and Pst1 and inserted into pIJ941 which has been digested with the same enzymes. Insertion at this point in the vector results in insertional inactivation of the hygromycin resistance gene. The ligation mixture was used to transform protoplasts of S. clavuligerus NTG 1. Thiostrepton resistant transformants which developed on regeneration agar were tested for hygromycin B sensitivity and then for IPNS activity. In order to test for IPNS production six hygromycin sensitive and one hygromycin resistant (control) colonies, were cultured, cell-free extracts were prepared and assayed for IPNS activity as outlined above. The control did not show a zone of inhibition of M. luteus indicator plates whereas all the the hygromycin sensitive colony enzyme extracts did, indicating that IPNS activity had been restored by introduction of this DNA fragment.

Although oreferred embodiments of the invention have been described herein in detail, it will be understood by those skilled in the art that variations may be made thereto without departing from the spirit of the invention or the scope of the appended claims.


**Claims**

1. A nucleotide sequence coding for the enzyme, isopenicillin N synthetase which is a protein produced by the metabolic system of Streptomyces clavuligerus.

2. A nucleotide sequence of claim 1 having a protein-coding region which includes .987 kb and codes for a protein having 329 amino acids.

3. A nucleotide sequence of claim 1 having the base sequence of Figure 1 exclusive of a stop codon TGA at the 988-990 positions.

4. A recombinant vector having said nucleotide sequence of claim 1.

5. A phage vector having said nucleotide sequence of claim 1.

6. A microorganism transformed with a recombinant plasmid of claim 3.

7. An amino acid sequence for the enzyme, isopenicillin N synthetase, as shown in Figure 1.

FIG.1.

## FIG.2.

```
ATGCCAGTTCTGATGCCGTCGGCCCACGTTCCGACCATCGACATCTCGCCGCTGTTCGGAACCGACGCCGCCGCGAAGAAGCGCGTCGCCGAGGAGATAC   100
 M  P  V  L  M  P  S  A  H  V  P  T  I  D  I  S  P  L  F  G  T  D  A  A  A  K  K  R  V  A  E  E  I  H

ACGGGGCCTGCCGCGGCTCGGGCTTCTTCTACGCCACGAACCACGGCGTGGACGTCCAGCAGCTCCAGGACGTGGTGAACGAGTTCCACGGCGCCATGAC   200
 G  A  C  R  G  S  G  F  F  Y  A  T  N  H  G  V  D  V  Q  Q  L  Q  D  V  V  N  E  F  H  G  A  M  T

CGACCAGGAGAAGCACGACCTGGCGATCCACGCGTACAACCCGGACAACCCGCATGTGCGCAACGGCTACTACAAGGCGGTCCCGGGCAGGAAGGCCGTC   300
 D  Q  E  K  H  D  L  A  I  H  A  Y  N  P  D  N  P  H  V  R  N  G  Y  Y  K  A  V  P  G  R  K  A  V

GAGTCCTTCTGTTACCTCAACCCGGACTTCGGCGAGGACCACCCGATGATCGCCGCGGGGACGCCGATGCACGAGGTGAACCTCTGGCCCGACGAGGAGC   400
 E  S  F  C  Y  L  N  P  D  F  G  E  D  H  P  M  I  A  A  G  T  P  M  H  E  V  N  L  W  P  D  E  E  R

GGCACCCGCGCTTCCGGCCGTTCTGCGAGGGCTACTACCGGCAGATGCTGAAGCTCTCCACCGTGCTCATGCGGGGGCTGGCGCTGGCGCTCGGGAGGCC   500
  H  P  R  F  R  P  F  C  E  G  Y  Y  R  Q  M  L  K  L  S  T  V  L  M  R  G  L  A  L  A  L  G  R  P

GGAGCACTTCTTCGACGCGGCGCTCGCCGAGCAGGACTCCCTGTCGTCCGTCTCGCTGATCCGCTACCCGTATCTGGAGGAGTACCCGCCGGTGAAGACG   600
 E  H  F  F  D  A  A  L  A  E  Q  D  S  L  S  S  V  S  L  I  R  Y  P  Y  L  E  E  Y  P  P  V  K  T

GGTCCCGACGGCCAGCTCCTGAGCTTCGAGGACCATCTGGACGTCTCGATGATCACCGTGCTCTTCCAGACCCAGGTGCAGAACCTCCAGGTGGAGACGG   700
 G  P  D  G  Q  L  L  S  F  E  D  H  L  D  V  S  M  I  T  V  L  F  Q  T  Q  V  Q  N  L  Q  V  E  T  V

TCGACGGCTGGCGGGACATCCCGACGTCGGAGAACGACTTCCTGGTCAACTGCGGTACCTACATGGCGCATGTCACGAACGACTACTTCCCGGCGCCCAA   800
 D  G  W  R  D  I  P  T  S  E  N  D  F  L  V  N  C  G  T  Y  M  A  H  V  T  N  D  Y  F  P  A  P  N

CCACCGGGTGAAGTTCGTGAACGCGGAGCGGCTGTCCCTGCCGTTCTTCCTCAACGGCGGGCACGAGGCGGTCATCGAGCCGTTCGTGCCGGAGGGCGCG   900
 H  R  V  K  F  V  N  A  E  R  L  S  L  P  F  F  L  N  G  G  H  E  A  V  I  E  P  F  V  P  E  G  A

AGCGAGGAGGTGAGGAACGAGGCCCTGTCCTACGGGGACTACCTCCAGCACGGGCTGCGGGCGCTGATCGTCAAGAACGGCCAGACCTGA   990
 S  E  E  V  R  N  E  A  L  S  Y  G  D  Y  L  Q  H  G  L  R  A  L  I  V  K  N  G  Q  T  X
```

EP 0 303 364 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | GENE, vol. 62, no. 2, February 1988, pages 187-196; B.K. LESKIW et al.: "Cloning and nucleotide sequence determination of the isopenicillin N synthetase gene from streptomyces clavuligerus" * whole document * | 1-7 | C 12 N 15/00 |
| D,Y | CAN. J. MICROBIOL., vol. 32, December 1986, pages 953-958, Ottawa, CN; S.E. JENSEN et al.: "Purification of isopenicillin N synthetase from streptomyces clavuligerus" * whole document * | 1-7 | |
| D,Y | NATURE, vol. 318, 14th November 1985, pages 191-194, London, GB; S.M. SAMSON et al.: "Isolation, sequence determination and expression in Esclenichia coli of the isopenicillin N synthetase gene from Cephalosporium acremonium" * whole document * | 1-7 | TECHNICAL FIELDS SEARCHED (Int. Cl.4) |
| P,A | EP-A-0 233 715 (BEECHAM GROUP PLC) * abstract; page 4, column 5, line 51 - column 6, line 6; page 3, column 3, lines 26-29; claims 1-4, 7, 10-11 * | 1-7 | C 12 N 15/00 C 12 N 9/00 |
| A | GENE, vol. 48, no. 2-3, 1986, pages 257-266; L.G. CARR et al.: "Cloning and expression of the isopenicillin N synthetase gene from Penicillin chrysogenum" * abstract; page 258 Materials and Methods; page 262, figure 4 * | 1-7 | |

-/-

The present search report has been drawn up for all claims

| Place of search | Date of completion of the search | Examiner |
|---|---|---|
| BERLIN | 12-10-1988 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                   

& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 200 425  (ELI LILLY AND CO.)<br>* abstract; page 2, column 2, lines 4-32; page 10, table T; claims 1, 4-5, 26, 29-30, 33-34 *<br>--- | 1 | |
| A | EP-A-0 225 128  (ELI LILLY AND CO.)<br>* whole document *<br>----- | 1 | |

TECHNICAL FIELDS
SEARCHED (Int. Cl.4)

The present search report has been drawn up for all claims

| Place of search | Date of completion of the search | Examiner |
|---|---|---|
| BERLIN | 12-10-1988 | JULIA P. |

EPO FORM 1503 03.82 (P0401)